# EUROPÄISCHE PATENTSCHRIFT

(11) **EP 1 167 358 B1**
(45) Veröffentlichungstag und Bekanntmachung des Hinweises auf die Patenterteilung: **15.09.2004**
(21) Anmeldenummer: 01115094.3
(22) Anmeldetag: 21.06.2001
(51) Int. Cl.: C07D 235/18, A61K 7/42

(54) **2-Phenylbenzimidazolsulfonsäuren als UV-B-Filter**
2-Phenylbenzimidazole sulphonic acids as UV-B filters
Acides 2-phenylbenzimidazole sulfoniques comme filtres UV-B

(30) Priorität: 23.06.2000 DE 10030663
(43) Veröffentlichungstag der Anmeldung: 02.01.2002
(73) Patentinhaber: MERCK PATENT GmbH, 64293 Darmstadt (DE)
(72) Erfinder: Heywang, Ulrich, Dr., 64289 Darmstadt (DE); Schwarz, Michael, Dr., 64331 Weiterstadt (DE); Pflücker, Frank, Dr., 64297 Darmstadt (DE)

(56) Entgegenhaltungen:
- EP-A- 0 669 323
- WO-A-93/15061
- DE-C- 676 103

## Beschreibung

Gegenstand der vorliegenden Erfindung sind 2-Phenylbenzimidazolsulfonsäuren, deren Verwendung als UV-Filter, ein Herstellverfahren für diese Verbindungen, sowie kosmetische Zubereitungen, die diese Verbindung enthalten.

Eine mehr oder minder stark ausgeprägte Sonnenbräune der Haut gilt in der modernen Gesellschaft als attraktiv und als Ausdruck von Dynamik und Sportlichkeit. Neben dieser erwünschten Wirkung der Sonne auf die Haut treten eine Reihe von unerwünschten Nebenwirkungen auf, wie Sonnenbrand oder vorzeitige Hautalterung und Faltenbildung. Inzwischen sind eine Anzahl von leistungsfähigen UV-Filtem entwickelt worden, die in Form von Cremes, Lotionen oder Gelen auf die Haut aufgetragen auch bei stärkerer Sonneneinwirkung die Entwicklung von Sonnenbrand wirksam verzögern können. Der in der pharmazeutischen oder kosmetischen Zubereitung enthaltene UV-Filter bildet auf der Oberfläche der Haut einen Film bzw. eine Schicht aus und dringt nicht mit weiteren in der Zubereitung enthaltenen pflegenden Substanzen in tiefere Hautschichten vor. Bekannte UV-Filter bzw. Sonnenschutzmittel wirken also nur in der Weise, dass sie bestimmte Bereiche des Sonnenlichts absorbieren und somit diese Strahlung nicht in tiefere Schichten der Haut vordringen kann. Bekanntlich wird der gefährlichste Teil der Sonnenstrahlung von den ultravioletten Strahlen mit einer Wellenlänge von weniger als 400 nm gebildet. Die untere Grenze der ultravioletten Strahlen, welche die Erdoberfläche erreichen, ist durch die Absorption in der Ozonschicht auf ca. 280 nm beschränkt. Die heute in der Kosmetik üblichen Sonnenschutzfilter absorbieren in einem Wellenlängenbereich von 280 bis 400 nm. Dieser Bereich umfasst UV-B-Strahlen mit einer Wellenlänge zwischen 280 und 320 nm, die bei der Bildung eines Sonnenerythems eine entscheidende Rollen spielen, wie auch UV-A-Strahlen, mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische und irritative Reaktionen auslösen können.

Aufgabe pflegender Kosmetik ist es, nach Möglichkeit den Eindruck einer jugendlichen Haut zu erhalten. Prinzipiell stehen verschiedene Wege offen, um diesen Weg zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen. Die Idee ist also, vorbeugend einzugreifen und dadurch den Alterungsprozess hinauszuzögern. Ein Beispiel sind hierfür die bereits erwähnten UV-Filter, welche durch Absorption bestimmter Wellenlängenbereiche eine Schädigung der Haut vermeiden oder zumindest vermindern. Entsprechend der Lage ihrer Absorptionsmaxima werden UV-Absorber für kosmetische und dermatologische Zubereitungen in UV-A- und UV-B-Absorber eingeteilt, wobei UV-A-Absorber üblicherweise auch im UV-B-Bereich absorbieren und daher alternativ auch als Breitbandabsorber oder -filter bezeichnet werden. Insbesondere bei der Vermeidung einer Hautschädigung durch Bildung von Sonnenerythemen kommt den UV-B-Filtern jedoch eine besondere Bedeutung zu, da Rezepturen auf Basis von Breitbandfiltern alleine hier nur ungenügenden Schutz bieten oder die erwünschte Bräunung der Haut ganz unterbinden. Daher besteht ständig ein Bedarf nach Substanzen mit einem Absorptionsmaximum im UV-B-Bereich, die sich gut in kosmetische Formulierungen einarbeiten lassen.

Für die Formulierung von entscheidender. Bedeutung ist die Löslichkeit der Filtersubstanzen in Öl- und Wasserphasen, da es insbesondere zur Einstellung eines hohen Schutzfaktors erforderlich ist, Filter in alle Phasen der Formulierung einzuarbeiten. Zu den öllöslichen UV-B-Filtern zählen Methoxyzimtsäureisooctylester, Methoxyzimtsäureisoamylester, und Methylbenzylidencampher. Unter den wasserlöslichen UV-B-Filtern sind insbesondere die Salze der 2-Phenylbenzimidazol-5-sulfonsäure zu nennen, deren Verwendung als UV-Strahlenfilter bereits im Deutschen Reichspatent Nr. 676 103 beschrieben wird.

Ein Nachteil dieser wasserlöslichen UV-B-Filter ist jedoch, dass die Formulierung nur im alkalischen Möglich ist, da die Sulfonsäure bei pH-Werten unter 7 ausfällt.

Daher bestand weiterhin Bedarf nach wasserlöslichen UV-B-Filtern, die sich gut zur Formulierung kosmetischer Rezepturen eignen.

Jetzt wurde überraschend gefunden, dass sich bestimmte 2-Phenylbenzimidazolsulfonsäuren bzw. deren Salze gut in kosmetische Rezepturen einarbeiten lassen, wobei die oben diskutierten Probleme nicht auftreten.

Ein erster Gegenstand der vorliegenden Erfindung sind dementsprechend 2-Phenylbenzimidazolsulfonsäuren gemäß Formel I 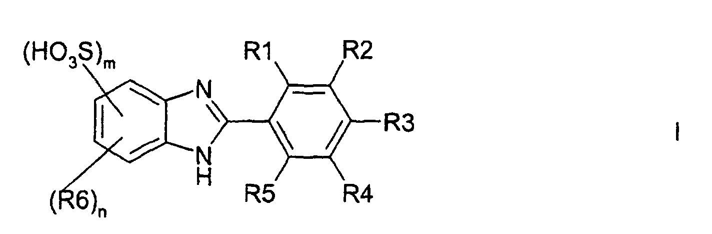 worin n 0, 1 oder 2 und m 2 oder 3 bedeutet, R1, R2, R3, R4 und R5 jeweils unabhängig voneinander stehen für einen Rest aus der Gruppe der H-, C₁₋₈-Alkyl-, C₁₋₈-Alkoxy-, Hydroxy-, Sulfat-, Nitro-, F-, Cl-, Br-, I-Reste und R6 ein C₁₋₈-Alkyl- oder C₁₋₈-Alkoxy-Rest ist.

Unter 2-Phenylbenzimidazolsulfonsäuren im Sinne der vorliegenden Erfindung werden dabei grundsätzlich auch die Salze dieser Säuren verstanden, wobei Salze vorzugsweise die Alkalimetallsalze, insbesondere die Natrium- oder Kaliumsalze, bzw. die Ammoniumsalze, insbesondere die Triethanolammoniumsalze der entsprechenden Sulfonsäuren sind.

Bei bevorzugten 2-Phenylbenzimidazolsulfonsäuren gemäß Formel I ist dabei m = 2 und n = 0 oder n = 1, wobei vorzugsweise mindestens 4 Reste aus der Gruppe R1, R2, R3, R4 und R5 H, insbesondere bevorzugt sogar alle Reste R1-R5 H sind. Erfindungsgemäß besonders bevorzugt ist dementsprechend die 2-Phenylbenzimidazol-4,6-disulfonsäure, die, wie in Formel la angedeutet, üblicherweise in Betainform vorliegt.

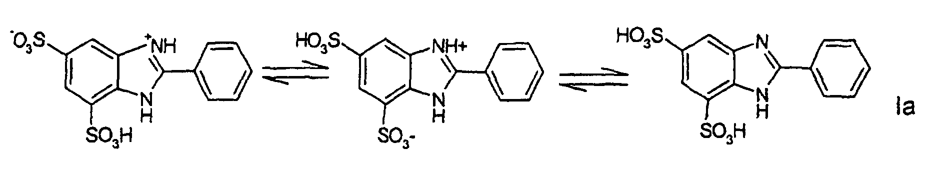

Die erfindungsgemäßen 2-Phenylbenzimidazolsulfonsäuren können nach jedem beliebigen dazu geeigneten Herstellverfahren erhalten werden. Als besonders gut geeignet und wirtschaftlich haben sich dabei Verfahren erwiesen, bei denen ein ortho-Phenylendiamin oder ein Derivat davon mit einer Arylcarbonsäure oder einem Arylcarbonsäurenderivat umgesetzt wird.

Ein zweiter Gegenstand der vorliegenden Erfindung ist daher ein Verfahren zur Herstellung der oben beschriebenen 2-Phenylbenzimidazolsulfonsäuren bei dessen Durchführung ein o-Phenylendiamin gemäß Formel II 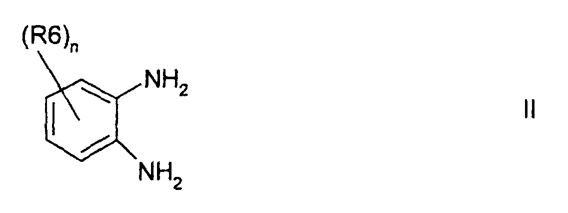 mit einer Verbindung gemäß Formel III 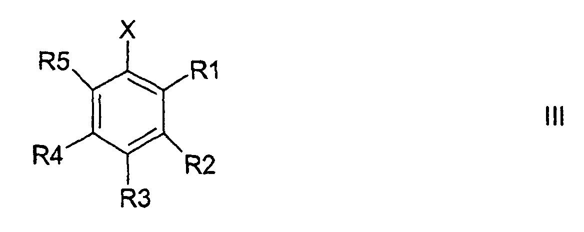 umgesetzt wird, wobei R1, R2, R3, R4 und R5 jeweils unabhängig voneinander stehen für einen Rest aus der Gruppe der H-, C₁₋₈-Alkyl-, C₁₋₈-Alkoxy-, Hydroxy-, Nitro-, F-, Cl-, Br-, l-Reste und X ausgewählt ist aus den Resten -COOH, -COCI, - COBr, -CN oder -COOR, wobei R ein C₁₋₂₀-Alkylrest ist.

In einer bevorzugten Ausführungsform des Verfahrens wird Schwefelsäure, insbesondere 96%ige Schwefelsäure als Lösungsmittel einsetzt. Dabei bewirkt schon die Schwefelsäure alleine bei geeigneter Temperatur eine Sulfonierung des Benzimidazols. So ist in der internationalen Anmeldung WO 93/15061 bereits ein Verfahren beschrieben, bei dem durch Umsetzung in Schwefelsäure monosulfonierte Produkte erhalten werden.

Im erfindungsgemäßen Verfahren zur Herstellung mehrfach sulfonierter Benzimidazole ist es jedoch bevorzugt, wenn eine aktivierte Schwefelsäure eingesetzt wird, wobei die Aktivierung vorzugsweise durch Chlorsulfonsäure oder Schwefeltrioxid erfolgt. Der Einsatz von Chlorsulfonsäure zur Herstellung von Bisbenzimidazoloylsulfonsäuren ist bereits in der Europäischen Patentanmeldung EP-A-669 323 beschrieben. Hinweise darauf, dass es unter Verwendung von Chlorsulfonsäure auch möglich ist, die erfindungsgemäßen Verbindungen herzustellen, enthält die Schrift jedoch nicht.

Erfolgt die Aktivierung durch Schwefeltrioxid, so wird üblicherweise die Oleum genannte "rauchende" Schwefelsäure eingesetzt, die eine Lösung von Schwefeltrioxid in konzentrierter Schwefelsäure darstellt. Dabei weist der Einsatz von Schwefeltrioxid (Oleum) zur Aktivierung der Schwefelsäure im Vergleich zum Einsatz von Chlorsulfonsäure verschiedene Vorteile auf:
- eine Gasentwicklung (HCI) während der Umsetzung erfolgt nicht, so dass eine Druckregelung nicht erforderlich ist,
- dementsprechend ist auch das Abfangen und Entsorgen des aggressiven Gases (HCI) nicht erforderlich,
- während für die anfallende chloridhaltige Schwefelsäure bei Verwendung von Chlorsulfonsäure extrem korrosionsbeständige Apparaturen erforderlich sind, kann mit Oleum in weniger aufwendigen Geräten gearbeitet werden,
- nach der Hydrolyse des Oleums liegt Schwefelsäure vor, die recycliert werden kann, während das Recycling der aus der Verwendung von Chlorsulfonsäure resultierenden chloridhaltigen Schwefelsäure nur schwer möglich ist.

Aufgrund dieser Vorteile ist es erfindungsgemäß besonders bevorzugt, wenn die Akivierung der Schwefelsäure durch Schwefeltrioxid (Oleum) erfolgt.

Wird das erfindungsgemäße Verfahren mit aktivierter Schwefelsäure durchgeführt, so ist es insbesondere bevorzugt, wenn X in Formel III ein Rest -COOH ist, d.h. wenn es sich bei Formel III um eine Arylcarbonsäure handelt. In einer anderen ebenfalls bevorzugten Variante des Verfahrens ist X in Formel III ein Rest -COOR, wobei R für einen C₁₋₂₀-Alkylrest, vorzugsweise einen C₁₋₈-Alkylrest und insbesondere bevorzugt einen Methyl- oder Ethylrest steht.

Insbesondere bevorzugt ist es dabei, wenn die Umsetzung bei Temperaturen zwischen 20°C und 200°C, vorzugsweise zwischen 160°C und 190°C erfolgt. Üblicherweise wird die Reaktionstemperatur für 2 bis 8 Stunden aufrecht erhalten; bei Reaktionszeiten von weniger als 2 Stunden werden noch Monosulfonierungsprodukte beobachtet, die sich vom Produkt nur schwer abtrennen lassen.

Die Durchführung des erfindungsgemäßen Verfahrens an sich ist einfach und bereitet dem Fachmann keinerlei Schwierigkeiten. Im folgenden ist eine bevorzugte Ausführungsform der Reaktionsdurchführung angegeben, die als Beispiel dienen kann, die möglichen Varianten der Reaktionsführung jedoch nicht einschränken soll:
Man legt die Schwefelsäure, vorzugsweise in Form einer 50-100%igen Lösung, insbesondere als konzentrierte ca. 96%ige Lösung vor und trägt das ortho-Phenylendiamin bzw. dessen Derivat darin ein. Anschließend erfolgt die Aktivierung mit Oleum (z.B. einer 65%ige Lösung von Schwefeltrioxid in Schwefelsäure), wobei die Temperatur vorzugsweise unter 150°C gehalten wird. Dabei wird die Menge an Schwefeltrioxid so gewählt, dass das gesamte, während der Reaktion freiwerdende Wasser abgefangen werden kann. D.h. zur Herstellung einer Benzimidazoldisulfonsäure werden pro mol o-Phenylendiamin mindestens 4 mol Schwefeltrioxid eingesetzt.
Die Zugabe des zweiten Reaktionspartners (Arylcarbonsäure bzw - derivat) erfolgt aus Sicherheitsgründen vorzugsweise erst nach Abkühlung auf eine Temperatur unterhalb 100°C, da sich die Temperatur der Reaktionsmischung durch die Zugabe gegebenenfalls weiter erhöht. Um erfindungsgemäße Produkte zu erhalten, erfolgt dabei der Einsatz von Arylcarbonsäure sinnvoll im Verhältnis 1:1 zu dem Phenylendiamin.
Anschließend wird das Reaktionsgemisch langsam auf Temperaturen zwischen 150 und 250 °C, vorzugsweise zwischen 160 und 200 °C erwärmt und 2 bis 8 Stunden bei dieser. Temperatur, gegebenenfalls unter Rühren, gehalten. Anschließend kühlt man die Reaktionsmischung, vorzugsweise auf Temperaturen unter 10°C ab und hydrolysiert in Wasser.
Nach kurzem Rühren, vorzugsweise 20 Minuten bis 2 Stunden, werden die festen Bestandteile abgetrennt, vorzugsweise mit warmen Wasser gewaschen und getrocknet.
Zur Reinigung des getrockneten Rohproduktes wird dieses vorzugsweise in Natronlauge gelöst und die so erhaltene Lösung, vorzugsweise mit Aktivkohle, gereinigt. Aus der farblosen Lösung wird das Endprodukt mittels einer Säure, vorzugsweise einer Mineralsäure, beispielsweise Schwefelsäure ausgefällt.

Aufgrund ihrer Absorptionmaxima im UV-B-Bereich eignen sich die erfindungsgemäßen 2-Phenylbenzimidazolsulfonsäuren als UV-B-Filtersubstanzen.

Dementsprechend ist ein weiterer Gegenstand der vorliegenden Erfindung die Verwendung einer erfindungsgemäßen 2-Phenylbenzimidazolsulfonsäure als UV-Filter, insbesondere als UV-B-Filter.

Aufgrund dieser Verwendungsmöglichkeit eignen sich die erfindungsgemäßen Substanzen hervorragend zum Einsatz in kosmetischen Zubereitungen. Ein weiterer Erfindungsgegenstand sind folglich kosmetische Zubereitungen mit UV-Schutz-Eigenschaften enthaltend mindestens eine erfindungsgemäße Verbindung nach Formel I.

Die schützende Wirkung dieser Zubereitungen gegen UV-Strahlung kann verbessert werden, wenn die Formulierung neben dem erfindungsgemäßen einen oder mehrere weitere UV-Filter enthält.

Prinzipiell kommen alle UV-Filter für eine Kombination in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.
- Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder α-(2-Oxoborn-3-yliden)toluol-4-sulfonsäure (z.B. Mexoryl® SL),
- Benzoyl- oder Dibenzoylmethane wie 1-(4-tert-Butylphenyl)-3-(4-methoxyphenyl)propan-1,3-dion (z.B. Eusolex® 9020) oder 4-lsopropyldibenzoylmethan,
- Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),
- Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),
- Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),
- 4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),
- Benzimidazolderivate wie 2-Phenylbenzimidazol-5-sulfonsäure sowie ihre Kalium-, Natrium-, Lithium-, Ammonium- und Triethanolaminsalze (z.B. Eusolex® 232), 2,2'-(1,4-Phenylen)bis-(1H-benzimidazol-4,6-disulfonsäure, Mononatriumsalz) (CAS-Nr. 180 898-37-7) und 2,2'-(1,4-Phenylen)bis-(1 H-benzimidazol-5-sulfonsäure) sowie ihre Kalium-, Natrium- und Triethanolaminsalze
- und weitere Substanzen wie 2-Cyano-3,3-diphenylacrylsäure-2-ethylhexylester (z.B. Eusolex® OCR), 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX), 2,4,6-Trianilino-(p-carbo-2'-ethylhexyl-1'-oxi)-1,3,5-triazin ( z.B. Uvinul® T 150), 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®), 4,4'-[(6-[4-((1,1-Dimethylethyl)aminocarbonyl)phenylamino]-1,3,5-triazin-2,4-diyl)diimino]bis-(benzoesäure-2-ethylhexylester) (z.B. Uvasorb® HEB), α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1), 2,2'-Methylen-bis-(6-(2H-benzotriazol-2-yl)-4-(1,1,3,3-tetramethylbutyl)-phenol) (CAS-Nr. 103 597-45-1), und 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6)..

Die in der Liste aufgeführten Verbindungen sind nur als Beispiele aufzufassen. Selbstverständlich können auch andere UV-Filter verwendet werden. Diese organischen UV-Filter werden wie auch die erfindungsgemäßen 2-Phenylbenzimidazolsulfonsäuren in der Regel in einer Menge von 0,5 bis 20 Gew.-%, vorzugsweise in einer Menge von 1 - 15 Gew.-% und insbesondere bevorzugt in Mengen von 2 bis 8 Gew.-% je Einzelsubstanz in kosmetische Formulierungen eingearbeitet. insgesamt enthalten die kosmetischen Zubereitungen üblicherweise bis zu 40 Gew.-%, vorzugsweise 5 bis 25 Gew.-% solcher organischer UV-Filter.

Als anorganische UV-Filter sind solche aus der Gruppe der Titandioxide wie z.B. gecoatetes Titandioxid (z.B. Eusolex® T-2000, Eusolex® T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Formulierungen eingearbeitet.

Werden verschiedene anorganische oder organische UV-Filter eingesetzt, so können diese in nahezu beliebigen Verhältnissen zueinander verwendet werden. Üblicherweise liegen die Verhältnisse der einzelnen Substanzen zueinander im Bereich 1:10 - 10:1, vorzugsweise im Bereich 1:5 - 5:1 und insbesondere bevorzugt im Bereich 1:2 - 2:1. Werden UV-A- neben UV-B-Filtern eingesetzt, so ist es für die meisten Anwendungen von Vorteil und daher erfindungsgemäß bevorzugt, wenn der Anteil an UV-B-Filtern überwiegt und das Verhältnis von UV-A-Filtern : UV-B-Filtern im Bereich 1:1 bis 1:3 liegt.

Neben den erfindungsgemäßen 2-Phenylbenzimidazolsulfonsäuren sind als bevorzugte Verbindungen mit UV-filternden Eigenschaften für die kosmetischen Zubereitungen 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, gecoatetes Titandioxid und insbesondere 2-Phenylbenzimidazol-5-sulfonsäure und 2,2'-(1,4-Phenylen)bis-(1H-benzimidazol-5-sulfonsäure) sowie ihre Kalium-, Natrium-, Lithium-, Ammonium- und Triethanolaminsalze zu nennen.

Die schützende Wirkung gegen oxidativen Stress bzw. gegen die Einwirkung von Radikalen kann weiter verbessert werden, wenn die Formulierung ein oder mehrere Antioxidantien enthält.

Es gibt viele aus der Fachliteratur bekannte und bewährte Substanzen, die verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutin bzw. Salze der Schwefelsäureester von Rutin und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercetin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Formulierungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004),

Die erfindungsgemäßen Formulierungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Formulierungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die erfindungsgemäßen Verbindungen der Formel I können in der üblichen Weise in kosmetische Formulierungen eingearbeitet werden. Geeignet sind Formulierungen für eine äußerliche Anwendung, beispielsweise als Creme, Lotion, Gel, oder als Lösung, die auf die Haut aufgesprüht werden kann. Dabei ist es bevorzugt, wenn die Zubereitung mindestens eine Öl- und mindestens eine Wasser-Phase enthält, wobei die erfindungsgemäße 2-Phenylbenzimidazolsulfonsäure in mindestens einer wässrigen Phase enthalten ist.

Als Anwendungsform der erfindungsgemäßen kosmetischen oder pharmazeutischen Formulierungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Formulierung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Alle Verbindungen oder Komponenten, die in den kosmetischen Formulierungen verwendet werden können, sind entweder bekannt und käuflich erwerbbar oder können nach bekannten Verfahren synthetisiert werden.

Die erfindungsgemäße kosmetische Zubereitung eignet sich besonders zum Schutz menschlicher Haut vor den schädigenden Einflüssen der UV-Anteile im Sonnenlicht, daneben bieten sie auch Schutz gegen Alterungsprozesse der haut sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form öligalkoholischer, ölig-wässriger oder wässrig-alkoholischer Gele bzw. Lösungen, als feste Stifte vorliegen oder als Aerosol konfektioniert sein.

Die Formulierung kann kosmetische Hilfsmittel enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den erfindungsgemäßen 2-Phenylbenzimidazolsulfonsäuren und vorzugsweise weiteren UV-Filtem beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße kosmetische Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpem.

Ist eine Zubereitung als Aerosol konfektioniert, verwendet man in der Regel die üblichen Treibmittel, wie Alkane, Fluoralkane und Chlorfluoralkane.

Die kosmetische Formulierung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Formulierung vor oder nach dem Shamponieren, vor oder nach dem Färben oder Entfärben bzw. vor oder nach der Dauerwelle aufgetragen wird. Es kann auch eine Formulierung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die kosmetische Formulierung kann außer der oder den erfindungsgemäßen 2-Phenylbenzimidazolsulfonsäuren und weiteren UV-Filtern verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäßen kosmetischen Zubereitungen können mit Hilfe von Techniken hergestellt werden, die dem Fachmann wohl bekannt sind.

Zum Schutz der Haut und/oder natürlicher oder sensibilisierter Haare vor Sonnenstrahlen wird auf die Haut oder die Haare eine kosmetische Zubereitung, enthaltend eine oder mehrere Verbindungen der Formel I aufgetragen. Als sensibilisierte Haare werden dabei Haare verstanden, welche einer chemischen Behandlung, wie einer Dauerwellenbehandlung, einem Färbe- oder Entfärbeprozeß unterzogen worden sind.

Ferner wirken Verbindungen der Formel I auch stabilisierend auf die Formulierung. Bei der Verwendung in entsprechenden Produkten bleiben diese daher auch länger stabil und verändern ihr Aussehen nicht. Insbesondere bleibt auch bei längerdauernder Anwendung bzw. längerer Lagerung die Wirksamkeit der Inhaltsstoffe, z.B. Vitamine, erhalten. Dies ist besonders vorteilhaft bei der Zusammensetzungen zum Schutz der Haut gegen die Einwirkung von UV-Strahlen, da diese Kosmetika besonders hohen Belastungen durch die UV-Strahlung ausgesetzt sind.

Ein weiterer Gegenstand der Erfindung betrifft die Stabilisierung von bestimmten UV-Filtern. Eine bekannte und leistungsfähige Klasse von Lichtschutzfiltersubstanzen wird von den Dibenzoylmethanderivaten gebildet. Nachteilig ist jedoch, daß diese Substanzen sehr leicht durch UV-Licht zersetzt werden und damit ihre schützenden Eigenschaften verlorengehen. Als Beispiel für einen auf dem Markt erhältlichen Lichtschutzfilter aus dieser Verbindungsklasse sei das 4-(tert.-Butyl)-4'-methoxydibenzoylmethan angeführt, welches die in Formel IV gezeigte Struktur aufweist.

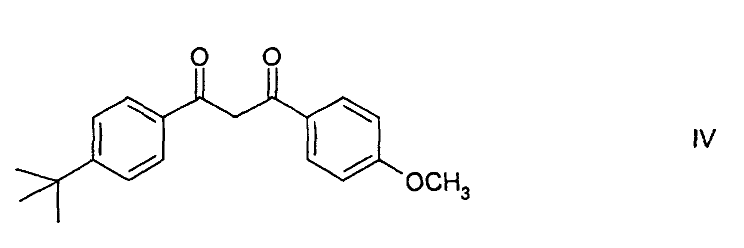

Überraschend hat sich nun gezeigt, daß Verbindungen mit der Formel I eine sehr gute Stabilisierungswirkung für die Dibenzoylmethane, insbesondere 4-(tert.buthyl)-4-methoxibenzoylmethan, aufweisen. Indem Gemische dieser Verbindungen in Kosmetika eingearbeitet werden, ist es nun möglich, Lichtschutzmittel unter Verwendung von Dibenzoylmethanen herzustellen, die auch bei längerer Sonneneinwirkung, beispielsweise während eines mehrstündigen Sonnenbades, keine oder nur eine geringe Verringerung der Schutzwirkung gegen UV-Strahlen aufweisen.

In den folgenden Beispielen wird die vorliegende Erfindung näher erläutert, ohne den Umfang der Erfindung zu beschränken. Dabei werden in den Beispielrezepturen die folgenden Handelsnamen verwendet:
**Antaron® V-220** wird von der Fa. GAF, Frechen, DE, vertrieben,
**Carbomer Ultrez-10** wird von der Fa. Goodrich, Neuss, DE, angeboten,
**Dehymuls®** E ist eine Mischung aus Dicocolypentaerytritylcitrat, Sorbitolsesquioleat, Bienenwachs und Aluminiumstearat und wird von der Fa. Cogni, Roermond, NL, vertrieben,
**Eusolex® 2292, Eusolex®232, Eusolex® 6300 und Eusolex® HMS** sind UV-Filter, die von der Merck KGaA, Darmstadt, DE, vertrieben werden,
**Luvitol® EHO** wird von der Fa. BASF AG, Ludwigshafen, DE, vertrieben,
**Pemulen® TR-1** und **Pemulen®TR-2** sind Acrylat/ Alkylacrylatpolymere, die von der Fa. Goodrich, Neuss, DE, vertrieben werden,
**Performa® V825** ist ein synthetisches Wachs, das von der Fa. New Phase, NJ08554, US, vertrieben wird,
**Oxynex® K** ist eine Mischung aus PEG-8, Tocophenol, Ascorbylpalmitat, Ascorbinsäure und Zitronensäure und wird von der Merck KGaA, Darmstadt, DE, vertrieben.

### Beispiel 1: Herstellung von 2-Phenylbenzimidazol-4,6-disulfonsäure

108 Teile o-Phenylendiamin werden in 500 Teile H₂SO₄ (> 96%) eingetragen und anschließend werden 800 Teile Oleum (65%ig) zugetropft, wobei die Temperatur bei maximal 120°C gehalten wird. Nach 15 min wird auf 70°C abgekühlt und es werden 120 Teile Benzoesäure zugegeben. Danach wird für 2 h auf 180°C erhitzt. Anschließend wird mit 2500 Teilen Wasser langsam hydrolysiert, wobei die Temperatur unter 10°C gehalten wird. Der Niederschlag (Kristalle) wird abgesaugt, das Rohprodukt in 8 Teilen Wasser suspendiert und mit 32 %iger Natronlauge bei pH = 7 gelöst. Anschließend wird mit Aktivkohle gerührt, bis die Lösung farblos ist, und danach mit 96 %iger H₂SO₄ durch Einstellung von pH = 1-2 gefällt. Es werden 300 Teile 2-Phenylbenzimidazol-4,6-disulfonsäure erhalten. Die Verbindung besitzt ein Absorptionsmaximum im UV-B-Bereich bei λₘₐₓ = 308 nm.

Analog werden hergestellt:
2-(4'-Methoxyphenyl)benzimidazol-4,6-disulfonsäure
2-(3'-Methoxyphenyl)benzimidazol-4,6-disulfonsäure
2-(4'-Ethoxyphenyl)benzimidazol-4,6-disulfonsäure
2-(3'-Ethoxyphenyl)benzimidazol-4,6-disulfonsäure
2-(3', 5'-Dimethoxyphenyl)benzimidazol-4,6-disulfonsäure
2-(3',5'-Diethoxyphenyl)benzimidazol-4,6-disulfonsäure
2-(3',4'-Diethoxyphenyl)benzimidazol-4,6-disulfonsäure.

### Beispiel 2: Sonnenschutzspray (O/W)

| Phase | Inhaltsstoff | **Gew.-%** |
|---|---|---|
| **A** | Eusolex®2292 (Art.-Nr. 105382) | 7,50 |
| | Eusolex®HMS (Art.-Nr. 111412) | 7,00 |
| | Steareth-2 | 0,40 |
| | Steareth-10 | 0,80 |
| | Pemulen® TR-2 | 0,18 |
| | Propylen-Glycol Isoceteth-3 Acetat | 5,00 |
| | Performa® V 825 | 0,80 |
| | Dimeticon | 1,00 |
| | Oxynex®K (Art.-Nr. 108324) | 0,10 |
| **B** | 2-Phenylbenzimidazol-4,6-disulfonsäure | 1,00 |
| | Triethanolamin | 0,90 |
| | Propandiol-1,2 | 2,00 |
| | Konservierungsmittel | 0,50 |
| | Wasser demineralisiert | a.d. 100,00 |

### Herstellung:

**Phase B:** Das Wasser wird mit dem Triethanolamin vermischt und anschließend die 2-Phenylbenzimidazol-4,6-disulfonsäure unter Rühren zugegeben. Sobald sich alles gelöst hat, werden die weiteren Bestandteile der Phase B zugegeben und anschließend die Mischung auf 80°C erwärmt.
**Phase A:** Die Bestandteile der Phase A mit Ausnahme von Permulen® TR-2 werden zusammengegeben und auf 80°C erwärmt. Anschließend wird das Permulen® TR-2 unter Rühren zugegeben.
**Herstellung des Sonnenschutzmittels:** Zur Phase A wird unter Rühren langsam die Phase B gegeben. Nach dem Homogenisieren wird unter Rühren abgekühlt.
Als Konservierungsmittel werden verwendet 0,05 % Propyl-4-hydroxybenzoat und 0,15 % Methyl-4-hydroxybenzoat.

### Beispiel 3: Sonnenschutzspray (O/W)

| Phase | Inhaltsstoff | **Gew.-%** |
|---|---|---|
| **A** | Eusolex® 2292 (Art.-Nr. 105382) | 7,50 |
| | Eusolex® HMS (Art.-Nr. 111412) | 7,00 |
| | Steareth-2 | 0,40 |
| | Steareth-10 | 0,80 |
| | Pemulen® TR-2 | 0,18 |
| | Propylen-Glycolisoceteth-3-acetat | 5,00 |
| | Performa® V 825 | 0,80 |
| | Dimeticon | 1,00 |
| | Oxynex® K (Art.-Nr. 108324) | 0,10 |
| **B** | 2-Phenylbenzimidazol-4,6-disulfonsäure | 1,00 |
| | Eusolex® 232 (Art.-Nr. 105372) | 1,00 |
| | Triethanolamin | 0,90 |
| | Propandiol-1,2 | 2,00 |
| | Wasser demineralisiert | a.d. 100,00 |

### Herstellung:

**Phase B:** Das Wasser wird mit dem Triethanolamin vermischt und anschließend Eusolex® 232 sowie die 2-Phenylbenzimidazol-4,6-disulfonsäure unter Rühren zugegeben. Sobald sich alles gelöst hat, werden die weiteren Bestandteile der Phase B zugegeben und anschließend die Mischung auf 80°C erwärmt.
**Phase A:** Die Bestandteile der Phase A mit Ausnahme von Permulen® TR-2 werden zusammengegeben und auf 80°C erwärmt. Anschließend wird das Permulen® TR-2 unter Rühren zugegeben.
**Herstellung des Sonnenschutzmittels:** Zur Phase A wird unter Rühren langsam die Phase B gegeben. Nach dem Homogenisieren wird unter Rühren abgekühlt.
Als Konservierungsmittel werden verwendet 0,05 % Propyl-4-hydroxybenzoat und 0,15 % Methyl-4-hydroxybenzoat.

### Beispiel 4: Sonnenschutzgel (wässrig)

| Phase | Inhaltsstoff | **Gew.-%** |
|---|---|---|
| **A** | 2-Phenylbenzimidazol-4,6-disulfonsäure | 1,00 |
| | Eusolex®232 (Art.-Nr. 105372) | 4,00 |
| | Natriumhydroxidlösung | 6,00 |
| | Glycerin | 3,00 |
| | Propandiol-1,2 | 2,00 |
| | Konservierungsmittel | q.s. |
| | Wasser demineralisiert | a.d. 100,00 |
| **B** | Carbomer Ultrez-10 | 0,70 |
| | Wasser, demineralisiert | 60,00 |
| **C** | Natriumhydroxidlösung (10 %) | 1,50 |
| | Wasser, demineralisiert | 4,00 |

### Herstellung:

Carbomer Ultrez-10 wird im Wasser der Phase B vollständig dispergiert. Anschließend wird langsam die Phase C zugegeben und homogenisiert.

Für die Phase A wird zunächst das Wasser zur Natriumhydroxidlösung gegeben und dann das Eusolex® 232 zugegeben und unter Rühren vollständig gelöst. Nach Erhalt einer klaren Lösung werden die weiteren Bestandteile der Phase A zugegeben. Anschließend wird Phase A portionsweise zum Gemisch der Phasen B und C gegeben, wobei nach jeder Zugabe homogenisiert wird.

Als Konservierungsmittel wird verwendet:
0,20 % Methyl-4-hydroxybenzoat

### Beispiel 5: Sonnenschutzgel (O/W)

| Phase | Inhaltsstoff | **Gew.-%** |
|---|---|---|
| **A** | Eusolex® 6300 (Art.-Nr. 5385) | 0,75 |
| | Luvitol® EHO | 10,00 |
| | Dimeticon | 2,00 |
| | Sheabutter | 5,00 |
| | Antaron® V-220 | 2,00 |
| | Oxynex® K | 1,00 |
| **B** | 2-Phenylbenzimidazol-4,6-disulfonsäure | 1,00 |
| | Eusolex® 232 (Art.-Nr. 105372) | 0,75 |
| | Tris(hydroxymethyl)-aminomethan | 0,33 |
| | Konservierungsmittel | q.s. |
| | Wasser demineralisiert | 20,00 |
| **C** | Tris(hydroxymethyl)-aminomethan | 1,20 |
| | Wasser, demineralisiert | 10,00 |
| **D** | Pemulen® TR-1 | 0,60 |
| | Wasser, demineralisiert | ad 100,00 |

### Herstellung:

Das Pemulen® TR-1 wird im Wasser der Phase D gelöst. Das Tris(hydroxymethyl)aminomethan wird im Wasser der Phase C gelöst und die Lösung zur Phase D gegeben. Das Tris(hydroxymethyl)aminomethan wird im Wasser der Phase B gelöst und dann unter Rühren das Eusolex® 232 zugegeben. Nach Erhalt einer klaren Lösung weden die weiteren Bestandteile der Phase B zugegeben und dann die Phase B zum Gemisch der Phasen C und D gegeben und homogenisiert. Die Bestandteile der Phase A werden vermengt und erwärmt. Dann wird Phase A unter Homogenisieren zum Gemisch der übrigen Phasen gegeben.
Als Konservierungsmittel werden verwendet 0,05 % Propyl-4-hydroxybenzoat und 0,15 % Methyl-4-hydroxybenzoat.

### Beispiel 6: Sonnenschutzgel (O/W)

| **Phase** | **Inhaltsstoff** | **Gew.-%** |
|---|---|---|
| **A** | Eusolex® 6300 (Art.-Nr. 5385) | 0,75 |
| | Luvitol® EHO | 10,00 |
| | Dimeticon | 2,00 |
| | Sheabutter | 5,00 |
| | Antaron® V-220 | 2,00 |
| | Oxynex® K liquid (Art.-Nr. 8324) | 1,00 |
| **B** | 2-Phenylbenzimidazol-4,6-disulfonsäure | 1,00 |
| | 2,2'-(1,4-Phenylen)bis-(1 H-benzimidazol-4,6-disulfonsäure) | 0,75 |
| | Tris(hydroxymethyl)-aminomethan | 0,33 |
| | Konservierungsmittel | q.s. |
| | Wasser demineralisiert | 20,00 |
| **C** | Tris(hydroxymethyl)-aminomethan | 1,20 |
| | Wasser, demineralisiert | 10,00 |
| **D** | Pemulen® TR-1 | 0,60 |
| | Wasser, demineralisiert | ad 100,00 |

### Herstellung:

Das Pemulen® TR-1 wird im Wasser der Phase D gelöst. Das Tris(hydroxymethyl)aminomethan wird im Wasser der Phase C gelöst und die Lösung zur Phase D gegeben. Das Tris(hydroxymethyl)aminomethan wird im Wasser der Phase B gelöst und dann unter Rühren die 2-Phenylbenzimidazol-4,6-disulfonsäure und die 2,2'-(1,4-Phenylen)bis-(1H-benzimidazol-4,6-disulfonsäure) zugegeben. Nach Erhalt einer klaren Lösung werden die weiteren Bestandteile der Phase B zugegeben und dann die Phase B zum Gemisch der Phasen C und D gegeben und homogenisiert. Die Bestandteile der Phase A werden vermengt und erwärmt. Dann wird Phase A unter Homogenisieren zum Gemisch der übrigen Phasen gegeben.
Als Konservierungsmittel wird verwendet 0,05 % Propyl-4-hydroxybenzoat und 0,15 % Methyl-4-hydroxybenzoat.

### Beispiel 7: Sonnenschutzlotion (W/O) mit UVA/B-Schutz

| Phase | Inhaltsstoff | **Gew.-%** |
|---|---|---|
| **A** | Eusolex® 2292 (Art.-Nr. 105382) | 3,00 |
| | Eusolex® 4360 (Art.-Nr. 1.05376) | 2,00 |
| | Dehymuls® E | 6,00 |
| | gehärtetes Castoröl | 1,00 |
| | Bienenwachs | 2,00 |
| | Oleylerucat | 6,00 |
| | Decycloeat | 6,00 |
| | Dimeticon | 1,00 |
| | Dicaprylether | 5,00 |
| **B** | Glycerin (87 %) | 5,00 |
| | 2-Phenylbenzimidazol-4,6-disulfonsäure | 3,00 |
| | Magnesiumsulfate Heptahydrat | 1,00 |
| | Konservierungsmittel | q.s. |
| | Wasser demineralisiert | a.d. 100,00 |

### Herstellung:

Die Bestandteile der Phasen A und B werden jeweils vermengt. Phase A wird auf 75°C und Phase B getrennt auf 80°C erwärmt. Phase B wird unter Homogenisieren zu Phase A gegeben. Anschließend wird unter Rühren abgekühlt.

Als Konservierungsmittel werden verwendet:
0,05 % Propyl-4-hydroxybenzoate
0,15 % Methyl-4-hydroxybenzoate.

### Beispiel 8: Sonnenschutzlotion (W/O) mit UVA/B-Schutz

| Phase | Inhaltsstoff | **Gew.-%** |
|---|---|---|
| **A** | Eusolex® 2292 (Art.-Nr. 105382) | 3,00 |
| | Eusolex® 4360 (Art.-Nr. 1.05376) | 2,00 |
| | Dehymuls® E | 6,00 |
| | gehärtetes Castoröl | 1,00 |
| | Bienenwachs | 2,00 |
| | Oleylerucat | 6,00 |
| | Decycloeat | 6,00 |
| | Dimeticon | 1,00 |
| | Dicaprylether | 5,00 |
| **B** | 2-Phenylbenzimidazol-4,6-disulfonsäure | 2,00 |
| | 2,2'-(1,4-Phenylen)bis-(1H-benzimidazol-5-sulfonsäure) | 2,00 |
| | Glycerin (87 %) | 5,00 |
| | Magnesiumsulfate Heptahydrat | 1,00 |
| | Konservierungsmittel | q.s. |
| | Wasser demineralisiert | a.d. 100,00 |

### Herstellung:

Die Bestandteile der Phasen A und B werden jeweils vermengt. Phase A wird auf 75°C erwärmt und getrennt davon Phase B auf 80°C. Phase B wird unter Homogenisierung zu Phase A gegeben. Anschließend wird unter Rühren abgekühlt.

Als Konservierungsmittel werden verwendet:
0,05 % Propyl-4-hydroxybenzoat
0,15 % Methyl-4-hydroxybenzoat.

## Patentansprüche

1. 2-Phenylbenzimidazolsulfonsäure gemäß Formel I 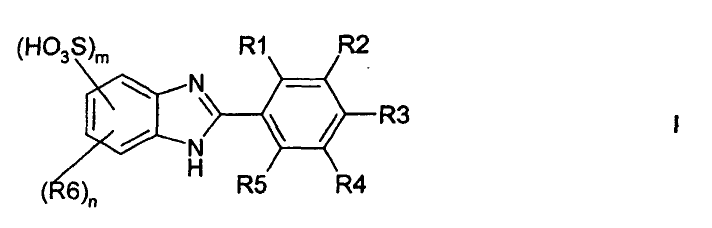 worin
n 0, 1 oder 2 und
m 2 oder 3 bedeutet,
R1, R2, R3, R4 und R5 jeweils unabhängig voneinander stehen für einen Rest aus der Gruppe der H-, C₁₋₈-Alkyl-, C₁₋₈-Alkoxy-, Hydroxy-, Sulfat-, Nitro-, F-, Cl-, Br-, I-Reste und
R6 ein C₁₋₈-Alkyl- oder C₁₋₈-Alkoxy-Rest ist.
und deren Salze.

2. 2-Phenylbenzimidazolsulfonsäure gemäß Anspruch 1, **dadurch gekennzeichnet, dass** m 2 und n 0 oder 1 ist, wobei vorzugsweise mindestens 4 Reste aus der Gruppe R1, R2, R3, R4 und R5 H, insbesondere bevorzugt sogar alle Reste R1-R5 H sind.

3. Verfahren zur Herstellung einer Verbindung gemäß einem der Ansprüche 1 bis 2, **dadurch gekennzeichnet, dass** ein o-Phenylendiamin gemäß Formel 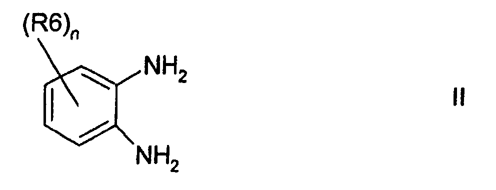 mit einer Verbindung gemäß Formel III 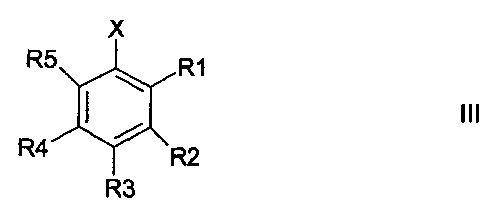 umgesetzt wird, wobei R1, R2, R3, R4 und R5 jeweils unabhängig voneinander stehen für einen Rest aus der Gruppe der H-, C₁₋₈-Alkyl-, C₁₋₈-Alkoxy-, Hydroxy-, Nitro-, F-, Cl-, Br-, I-Reste und X ausgewählt ist aus den Resten -COOH, -COCI, -COBr, -CN oder -COOR, wobei R für einen C₁₋₂₀-Alkylrest steht.

4. Verfahren nach Anspruch 3, **dadurch gekennzeichnet, dass** die Umsetzung in Schwefelsäure, insbesondere in aktivierter Schwefelsäure bei Temperaturen zwischen 20°C und 200°C, vorzugsweise zwischen 160°C und 190°C erfolgt, wobei die Aktivierung vorzugsweise durch Chlorsulfonsäure oder Schwefeltrioxid (Oleum), insbesondere bevorzugt durch Schwefeltrioxid (Oleum) erfolgt.

5. Verwendung einer 2-Phenylbenzimidazolsulfonsäure nach einem der Ansprüche 1 oder 2 als UV-Filter.

6. Kosmetische Zubereitung mit UV-Schutz-Eigenschaften enthaltend mindestens eine 2-Phenylbenzimidazolsulfonsäure nach einem der Ansprüche 1 oder 2.

7. Kosmetische Zubereitung nach Anspruch 6, **dadurch gekennzeichnet, dass** sie mindestens eine Öl- und mindestens eine Wasser-Phase enthält, wobei die 2-Phenylbenzimidazolsulfonsäure nach einem der Ansprüche 1 oder 2 in mindestens einer wässrigen Phase enthalten ist.

8. Kosmetische Zubereitung nach Anspruch 6 oder 7, **dadurch gekennzeichnet, dass** sie neben der 2-Phenylbenzimidazolsulfonsäure nach einem der Ansprüche 1 oder 2 noch mindestens eine weitere UV-Filter-Substanz enthält, die vorzugsweise ein UV-A-Filter ist, wobei die Zubereitung die einzelnen Filtersubstanzen in einer Menge von 0,5 bis 20 Gew.-%, vorzugsweise in einer Menge von 1 - 15 Gew.-% und insbesondere bevorzugt in einer Menge von 2 bis 8 Gew.-% enthält und insgesamt bis zu 40 Gew.-%, vorzugsweise 5 bis 25 Gew.-% solcher UV-Filter enthält.

9. Kosmetische Zubereitung nach einem der Ansprüche 6 bis 8, **dadurch gekennzeichnet, dass** die Zubereitung neben den erfindungsgemäßen 2-Phenylbenzimidazolsulfonsäuren mindestens eine Verbindung aus der Gruppe 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, gecoatetes Titandioxid, 2-Phenylbenzimidazol-5-sulfonsäure und 2,2'-(1,4-Phenylen)bis-(1H-benzimidazol-5-sulfonsäure), insbesondere bevorzugt jedoch 2-Phenylbenzimidazol-5-sulfonsäure und/oder 2,2'-(1,4-Phenylen)bis-(1H-benzimidazol-5-sulfonsäure) enthält.

10. Verwendung einer 2-Phenylbenzimidazolsulfonsäure nach einem der Ansprüche 1 oder 2 zur Stabilisierung von UV-Filtem.

## Claims

1. 2-Phenylbenzimidazolesulfonic acid according to formula I 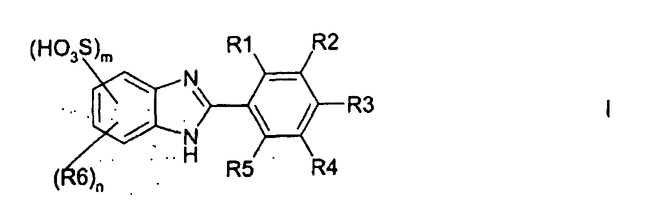 in which
n is 0, 1 or 2 and
m is 2 or 3,
R1, R2, R3, R4 and R5, in each case independently of one another, are a radical from the group H, C₁₋₈-alkyl, C₁₋₈-alkoxy, hydroxyl, sulfate, nitro, F, Cl, Br or I radicals, and
R6 is a C₁₋₈-alkyl or C₁₋₈-alkoxy radical,
and the salts thereof.

2. 2-Phenylbenzimidazolesulfonic acid according to Claim 1, **characterized in that** m is 2 and n is 0 or 1, where preferably at least 4 radicals from the group R1, R2, R3, R4 and R5 are H, and particularly preferably even all of the radicals R1-R5 are H.

3. Process for the preparation of a compound according to one of Claims 1 to 2, **characterized in that** an o-phenylenediamine according to formula II 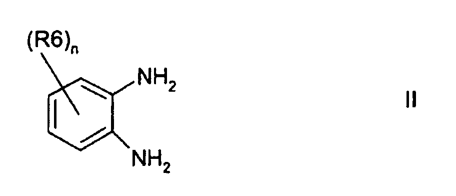 is reacted with a compound according to formula III 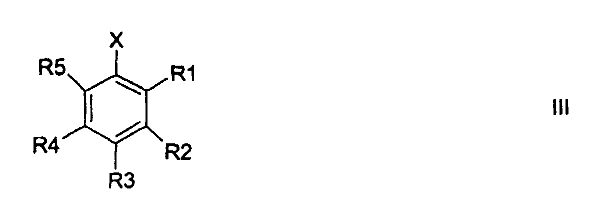 where R1, R2, R3, R4 and R5, in each case independently of one another, are a radical from the group of H, C₁₋₈-alkyl, C₁₋₈-alkoxy, hydroxyl, nitro, F, Cl, Br or I radicals, and X is chosen from the radicals -COOH, -COCl, -COBr, -CN or - COOR, where R is a C₁₋₂₀-alkyl radical.

4. Process according to Claim 3, **characterized in that** the reaction is carried out in sulfuric acid, in particular in activated sulfuric acid, at temperatures between 20°C and 200°C, preferably between 160°C and 190°C, the activation preferably being carried out by chlorosulfonic acid or sulfur trioxide (oleum), particularly preferably by sulfur trioxide (oleum).

5. Use of a 2-phenylbenzimidazolesulfonic acid according to one of Claims 1 or 2 as UV filter.

6. Cosmetic preparation having UV protection properties comprising at least one 2-phenylbenzimidazolesulfonic acid according to one of Claims 1 or 2.

7. Cosmetic preparation according to Claim 6, **characterized in that** it comprises at least one oil phase and at least one water phase, the 2-phenylbenzimidazolesulfonic acid according to one of Claims 1 or 2 being present in at least one aqueous phase.

8. Cosmetic preparation according to Claim 6 or 7, **characterized in that**, in addition to the 2-phenylbenzimidazolesulfonic acid according to one of Claims 1 or 2, it also comprises at least one further UV filter substance, which is preferably a UV-A filter, the preparation comprising the individual filter substances in an amount of from 0.5 to 20% by weight, preferably in an amount of from 1-15% by weight and particularly preferably in an amount of from 2 to 8% by weight, and overall comprises up to 40% by weight, preferably 5 to 25% by weight, of such UV filters.

9. Cosmetic preparation according to one of Claims 6 to 8, **characterized in that** the preparation, in addition to the 2-phenylbenzimidazolesulfonic acids according to the invention, comprises at least one compound from the group consisting of 3-(4'-methylbenzylidene)-dl-camphor, 1-(4-tert-butylphenyl)-3-(4-methoxyphenyl)propane-1,3-dione, 4-isopropyldibenzoylmethane, 2-hydroxy-4-methoxybenzophenone, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, coated titanium dioxide, 2-phenylbenzimidazole-5-sulfonic acid and 2,2'-(1,4-phenylene)bis(1H-benzimidazole-5-sulfonic acid), but particularly preferably 2-phenylbenzimidazole-5-sulfonic acid and/or 2,2'-(1,4-phenylene)bis(1H-benzimidazole-5-sulfonic acid).

10. Use of a 2-phenylbenzimidazolesulfonic acid according to one of Claims 1 or 2 for the stabilization of UV filters.

## Revendications

1. Les acides 2-phénylbenzimidazolsulfoniques selon la formule I 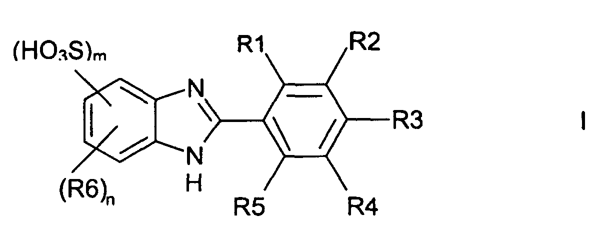 où
n est égal à 0, 1 ou 2 et
m à 2 ou 3,
R1, R2, R3, R4 et R5 représentent, indépendamment les uns des autres, un reste du groupe constitué par les restes H, alkyles en C₁₋₈, alcoxy en C₁₋₈, hydroxy, sulfate, nitro, F, Cl, Br, I et
R6 est un reste alkyle en C₁₋₈ ou un reste alcoxy en C₁₋₈,
et leurs sels.

2. Les acides 2-phénylbenzimidazolsulfoniques selon la revendication 1, **caractérisés en ce que** m est égal à 2 et n à 0 ou 1, de préférence, au moins quatre restes du groupe R1, R2, R3, R4 et R5 étant l'hydrogène, et même, de préférence, tous les restes R1 à R5 étant l'hydrogène.

3. Procédé pour la préparation d'un composé selon la revendication 1 ou 2, **caractérisé en ce que** l'on fait réagir une o-phénylènediamine selon la formule II 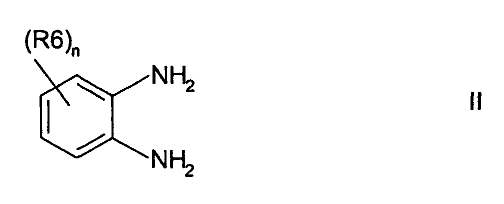 avec un composé selon la formule III 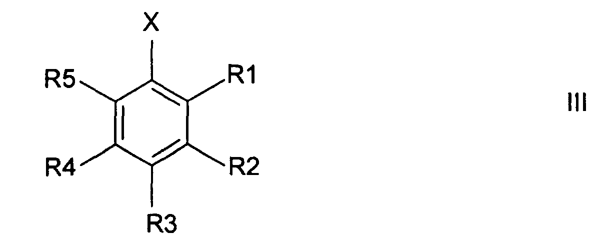 R1, R2, R3, R4 et R5 représentent, indépendamment les uns des autres, un reste du groupe constitué par les restes H, alkyles en C₁₋₈, alcoxy en C₁₋₈, hydroxy, nitro, F, CI, Br, I et X étant choisi parmi les restes -COOH, -COCl, - COBr, -CN ou -COOR, R représentant un reste alkyle en C₁₋₂₀.

4. Procédé selon la revendication 3, **caractérisé en ce que** la réaction est effectuée dans l'acide sulfurique, en particulier dans l'acide sulfurique activé, à des températures comprises entre 20°C et 200°C, de préférence entre 160°C et 190°C, l'activation étant réalisée de préférence par l'acide chlorosulfonique ou par l'anhydride sulfurique (oléum), en particulier, de préférence, par l'anhydride sulfurique (oléum).

5. Utilisation d'un acide 2-phénylbenzimidazolsulfonique selon la revendication 1 ou 2 comme filtre UV.

6. Préparation cosmétique présentant des propriétés de protection contre les UV contenant au moins un acide 2-phénylbenzimidazolsulfonique selon la revendication 1 ou 2.

7. Préparation cosmétique selon la revendication 6, **caractérisée en ce qu'**elle contient au moins une phase huileuse et au moins une phase aqueuse, l'acide 2-phénylbenzimidazolsulfonique selon la revendication 1 ou 2 étant contenu dans au moins une phase aqueuse.

8. Préparation cosmétique selon la revendication 6 ou 7, **caractérisée en ce qu'**elle contient à côté de l'acide 2-phénylbenzimidazolsulfonique selon la revendication 1 ou 2 au moins une autre substance filtrant les UV, ladite substance étant, de préférence, un filtre UVA, la préparation contenant les différentes substances filtrantes en une quantité comprise entre 0,5 et 20 % en poids, de préférence en une quantité comprise entre 1 et 15 % en poids et plus particulièrement en une quantité comprise entre 2 et 8 % en poids et au total jusqu'à 40 % en poids, de préférence 5 à 25 % en poids de tels filtres UV.

9. Préparation cosmétique selon l'une des revendications 6 à 8, **caractérisée en ce que** la préparation contient à côté des acides 2-phénylbenzimidazolsulfoniques conformes à l'invention au moins un composé du groupe constitué par le 3-(4'-méthylbenzylidène)-d1-camphre, la 1-(4-tert-butylphényl)-3-(4-méthoxyphényl)propane-1,3-dione, le 4-isopropyldibenzoyl-méthane, la 2-hydroxy-4-méthoxybenzophénone, l'ester octylique de l'acide méthoxycinnamique, le salicylate de 3,3,5-triméthylcyclohexyle, l'ester 2-éthylhexylique de l'acide 4-(diméthylamino)benzoïque, l'ester 2-éthylhexylique de l'acide 2-cyano-3,3-diphénylacrylique, le dioxyde de titane enrobé, l'acide 2-phénylbenzimidazol-5-sulfonique et l'acide 2,2'-(1,4-phénylène)bis-(1H-benzimidazol-5-sulfonique), de préférence toutefois l'acide 2-phénylbenzimidazol-5-sulfonique et/ou l'acide 2,2'-(1,4-phénylène)bis-(1H-benzimidazol-5-sulfonique).

10. Utilisation d'un acide 2-phénylbenzimidazolsulfonique selon la revendication 1 ou 2 pour la stabilisation des filtres UV.
